Europäisches Patentamt

European Patent Office

Office européen des brevets

(11) Publication number: **0 125 118 B1**

# EUROPEAN PATENT SPECIFICATION

(45) Date of publication of patent specification: **16.09.92**

(51) Int. Cl.5: **G01N 33/52**, G01N 33/74, G01N 33/76, G01N 33/535, G01N 33/543

(21) Application number: **84303021.4**

(22) Date of filing: **04.05.84**

The file contains technical information submitted after the application was filed and not included in this specification

(54) **Colorimetric immunoassay on solid surface, dipstick therefor and its preparation.**

(30) Priority: **06.05.83 US 492400**

(43) Date of publication of application:
**14.11.84 Bulletin 84/46**

(45) Publication of the grant of the patent:
**16.09.92 Bulletin 92/38**

(84) Designated Contracting States:
**AT BE CH DE FR GB IT LI LU NL SE**

(56) References cited:
EP-A- 0 063 810       EP-A- 0 103 958
EP-A- 0 124 124       WO-A-80/02800
DE-A- 3 346 795       US-A- 4 168 146
US-A- 4 299 916       US-A- 4 391 904

CLINICAL CHEMISTRY, vol.28, no.9, 1982,
Washington, DC (US); H.G.WADA et al.,
pp.1862-1866

(73) Proprietor: **OUIDEL CORPORATION**
**10165 McKellar Court**
**San Diego, California 92121(US)**

(72) Inventor: **Wada, Henry Garrett**
**33454 Caliban Drive**
**Fremont, CA 94536(US)**
Inventor: **Baxter, Sarah R.**
**5453 Muir Drive**
**San José, CA 95124(US)**
Inventor: **Rundle, Douglas D.**
**817 Lincoln Avenue**
**Palo Alto, CA 94301(US)**
Inventor: **Kasper, Kenneth C.**
**580 South Frances**
**Sunnyvale, CA 94086(US)**
Inventor: **Lankford, John C.**
**12200 Winton Way**
**Los Altos Hills, CA 94022(US)**

(74) Representative: **Allard, Susan Joyce et al**
**BOULT, WADE & TENNANT, 27 Furnival Street**
**London EC4A 1PO(GB)**

Rank Xerox (UK) Business Services

**Description**

The present invention relates to an immunoassay using an enzyme label, in which the detection of the unknown substance is performed colorimetrically directly upon a solid surface. More specifically, the system is related to the detection of antigen or antibody in a biologically derived fluid sample, such as serum or urine.

There are a number of known techniques for the determination of an unknown quantity of antigen or antibody in a biological fluid, such as serum or urine. Two widely used ones are competitive binding and sandwich immunoassays utilizing labeled or tagged immunochemical reagents. For example, in a typical sandwich technique for the detection of unknown antigen in a sample, antibody for the unknown antigen is bound to a solid surface and reacted with the antigen. After washing of the solid surface, a labeled antibody is reacted with the surface and the amount of bound labeled antibody is measured as an indication of the antigen quantity in the serum sample. In the competitive technique, antibody bound to a solid surface may be contacted with the sample containing an unknown quantity of the antigen to be determined, and with labeled antigen of the same type. After washing, the amount of labeled antigen bound on the surface is measured, providing an indirect indication of the amount of unknown antigen in the sample. Known labels are of the radioactive or fluorometric type, which are detected by instrumentation, and colorimetric labels, typically an enzyme label which causes the conversion of a corresponding substrate to colored form.

In one competitive technique, antigen is determined with enzyme-labeled antigen and an antibody-coated tube. [Engvall, et al, Biochim. Biophys. Acta. 251 (1971), 427-434.] There, antibody bound to the test tube wall is contacted with the fluid sample containing the unknown antigen, and with a known quantity of labeled antigen to form an immunological reaction product. After washing, the tube is filled with a solution containing a substrate which changes color in the presence of the enzyme label on the antigen. A chemical stop agent is added to terminate the reaction, and the substrate solution with the substrate in soluble form is placed in a microcuvette. The color change of the substrate in the solution is determined by measuring the absorbence of the solution at a particular wavelength. This system suffers from certain disadvantages, including the requirement of using an independent detection instrument, no permanent record of the test results, the requirement of a chemical stop solution for the enzymatic reaction, and inaccuracies due to unbound labeled antigen on the surface washing into the substrate solution and converting the substrate.

In another immunoassay, the labeled substance is deposited onto a surface which is read directly. In Harte U.S. Patent 4,133,639, the main system disclosed is that of depositing a fluorochrome labeled substance onto an impermeable continuous surface, and reading the surface with a fluorometer. The system is also stated to be applicable to detection of radioactive labels in a radioimmunoassay, and to spectrophotometric detection. In addition, the patent suggests at column 7, lines 25-38, that the system is also applicable to the use of enzyme-labeled systems such as disclosed in Pesce et al, Clin. Chem. 20/3, 353-359 (1974). In that system, the color is developed by converting the soluble substrate to a colored form, and reading the color spectrophotometrically in the solution. The Harte patent states that it differs from Pesce in that the diagnostic reagent is adsorbed (rather than covalently bonded) to a test tube support. Although a major thrust of the Harte patent is the direct reading of a surface with an instrument, there is no suggestion in Harte how the Pesce system could be adapted to direct reading of the surface.

US-A-4168146 discloses a bibulous test strip for carrying out immunoassays, the strip having antibodies bound to it. The strip in use is contacted with the aqueous solution containing a suspected antigen. It is then contacted in turn with an aqueous solution containing labeled antibodies.

EP-A-0063810 discloses devices and kits for immunoassays which comprise solid porous supports, preferably in the form of a sheet, with antigens or immunoglobulins, or both, bound directly to them.

US-A-4299916 discloses an assay method which utilises a solid surface to which one of the members of an immunological pair (mip) is bonded, and a signal producing system, which includes a catalytic member bonded to a mip, the signal system resulting in a measurable signal on said solid surface which is related to the amount of analyte in the medium.

WO-A-8002800 discloses a method for detecting ovulation in human females comprising the use of a bibulous mat which contains an antibody against estrogen-induced peroxidase.

Clinical Chemistry vol. 28, no. 9 (1982), page 1862 to 1866 discloses enzyme immunoassay techniques for a range of glycoprotein tropic hormones utilising antibodies to the $\alpha$ and $\beta$ subunits of the hormone to be assayed. The anti-$\alpha$-subunit antibody is coupled to horseradish peroxidase.

According to the present invention, a colorimetric immunoassay of antigen or antibody is provided by direct visual observation of a color upon a solid surface. Referring to a sandwich immunoassay for detecting antigen in a fluid sample, sample containing unknown antigen is contacted with a solid phase comprising immobilized antibody bound in a layer to a solid surface, and with a soluble enzyme-labeled antibody. The antigen immunologically bonds to the immobilized antibody and to the labeled antibody. The solid phase is washed free of unreacted soluble enzyme-labeled antibody. Then, the solid phase is contacted with a solution containing soluble substrate for the enzyme to cause the substrate to be converted into an insoluble colored form which deposits on the solid phase. Thereafter, the solid phase is separated from the substrate solution and is visually read as a semiquantitative indication of the presence of the antigen to be detected. The system is also applicable to the detection of unknown antibody in an analogous sandwich technique. A preferred solid surface is non-swellable impermeable plastics continuous surface of a dipstick, although other forms of the solid surfaces such as a bead may be employed. This system is also applicable to a competitive colorimetric immunoassay. The system is applicable to the detection of pregnancy, wherein the antigen to be detected is human chorionic gonadotropin, and to the detection of ovulation wherein the antigen is luteinizing hormone.

The following advantages are achieved by use of the present system. Increased sensitivity is provided because of the enzyme-substrate amplification of the signal in contrast to direct labeling of the antibody or antigen. Background noise due to incomplete washing of the enzyme label is minimized by direct reading of the solid surface, rather than reading the substrate solution, which may include unbound enzyme labeled substance which has not been removed by washing. Also, termination of the enzymatic reaction on the dipstick is readily accomplished by removal from the substrate solution and rinsing in water and, optionally, blotting, in contrast to the requirement of a chemical stop solution where the soluble substrate is read in solution. In addition, the colored dipstick can be used as a permanent record of test results. Semi-quantitation of the test results is achieved by visual inspection of the intensity of the color on the stick, in comparison with a color chart or reference stick. Furthermore, the system does not require an instrument and so can be used for the home test market. Regarding such market, the colorimetric end point is more easily read than other systems which do not require instrumentation, such as agglutination reaction tests.

The system utilizes conventional sandwich or competitive techniques. Enzyme-labeled diagnostic reagent is immunologically bound to a solid surface, and the enzyme interacts with a soluble substrate to cause it to be converted into an insoluble colored form, which deposits as a precipitate onto the solid phase. The solid phase is removed from the reaction vessel and substrate and, optionally, is washed. The amount of unknown substance in the sample can be determined by viewing the color directly on the surface of the solid phase.

Thus, in general the invention provides a method for the colorimetric immunoassay of an immunologically reactive substance to be detected in a fluid sample, comprising:

contacting said sample with a non-swellable impermeable plastics continuous solid surface, whereby the chemical nature of the plastic surface is such as to permit binding of an antigen or an antibody to it, adapted to immobilize substance to be detected thereon from said sample and using said surface to immobilize an enzyme labeled diagnostic reagent for said substance to be detected in an amount related to the amount of said substance which has been immobilized, contacting the resulting solid material with a solution of a substrate for said enzyme which is converted thereby into an insoluble colored form and allowing said insoluble colored form to deposit on said resulting impermeable solid material as a precipitate to impart thereto a visually discernible color related to the presence of said substance, and separating the resulting colored solid material from said substrate solution.

A general aspect of the invention provides such a method comprising

(a) contacting said sample containing said substance to be detected with an impermeable solid phase comprising immobilized anti-substance bound in a layer to said solid surface, and with either soluble enzyme-labeled anti-substance or with free enzyme-labeled substance of the same type as said substance to be detected thereby to cause an amount of the enzyme label to be immobilized which is related to the amount of said substance to be detected in the sample;

(b) separating the resulting solid phase from step (a) from liquid phase;

(c) contacting the separated solid phase from step (b) with a solution containing soluble substrate for the enzyme to cause the substrate to be converted into an insoluble colored form which deposits as a precipitate onto the impermeable solid phase to impart a visually discernible color to it related to the presence of said substance to be detected in the sample; and

(d) separating said solid phase from said substrate solution.

For simplicity of description, the system will first be described with respect to a sandwich-type colorimetric immunoassay of antigen. An effective sandwich methodology using enzyme labels is set out in Wada, H. G., et al, Clin. Chem. 28/9, 1862-1866. The above sandwich system has been described for the detection of antigen in a biologically derived sample.

In the sandwich technique, the antibody is immobilized as a bound layer on a solid surface, such as the reaction portion of a dipstick. This solid phase is then contacted with the fluid sample containing antigen, and with enzyme-labeled antibody immunologically reactive with the antigen. The antigen binds to the immobilized antibody on the solid phase, and with the labeled antibody, also on the solid phase, to form a reaction product in order comprising solid surface* antibody*antigen*enzyme-labeled-antibody. The "*" signifies a bond. For example, the bond between the surface and antibody may be a covalent bond or an adsorption bond. The bonds between the immobilized antibody and the antigen, and between the antigen and the labeled antibody, comprise immunological bonds. After formation of the reaction product, the solid phase is separated from the unreacted soluble enzyme-labeled antibody and, preferably, is washed. Then, the solid phase is reacted with a solution containing soluble substrate for the enzyme to cause the substrate to be converted into insoluble colored form, which deposits on the solid phase to impart a visually discernible color to it. The surface is removed from the substrate and the color is observed as an indication of the quantity of antigen in the sample. The surface may be blotted or rinsed with water to remove residual substrate for complete termination of the enzymatic reaction and storage of the reacted dipstick.

The solid surface to be layered with the antigen or antibody comprises a non-swellable, impermeable plastics continuous surface. The term "impermeable" is intended to exclude a surface capable of substantial retentive absorption of reagents during washing, such as filter paper. In other words, the solid surface should be sufficiently impermeable to liquid to permit effective removal by washing of unbound reagent. A preferred color for the dipstick is white or other color that enhances the color of the dye. If desired, solid phases other than dipsticks, such as the solid surface of a bead, may also be employed.

The chemical nature of the surface to be layered is such as to permit binding of the antigen or antibody. For covalent binding, suitable surfaces include a plastic such as polymethylmethacrylate, polystyrene, polyethylene poly-terephthalate, polyester, polypropylene, and the like. In a typical instance, the diagnostic reagent (antigen or antibody) nay be bound to a plastic sheet which is thereafter cut to an appropriate dipstick shape that will fit into a reaction vessel such as a test tube. The term "dipstick" means an article with a handle portion and a reaction surface portion. For covalent bonding, indirect coupling may be used in which coupling reagents reactive with functional groups of the diagnostic reagents serve as bridges between the polymer surface and a diagnostic reagent. Such functional groups include amino groups, hydroxyl groups, mercapto groups, amido groups, and carboxyl groups. Suitable coupling techniques between the polymer and diagnostic reagent are set forth in Bennich et al U.S. patent 3,720,760. In some instances, the diagnostic reagent to be immobilized may be coated by adsorption onto the solid surface rather than being covalently bonded.

In a specific technique, covalent bonding of antibody to the dipstick may be accomplished by pre-coating the plastic surface with a film of agarose or other hydrophilic polymer such as polysaccharide, polyethylene glycol, polypeptide, and the like. The antibody or antigen is then covalently attached to this hydrophilic coating. The hydrophilic coating avoids non-specific binding of the analyte or enzyme-conjugate to the plastic surface by hydrophobic interaction. Thus, use of this type of dipstick minimizes background binding.

In the next step, the immobilized antibody-containing solid phase is contacted with the biologically derived fluid sample, typically serum or urine, containing the unknown antigen to be detected, and with soluble enzyme-labeled antibody. The immobilized antibody and enzyme-labeled antibody are both immunologically reacted with the antigen so that the reaction product is formed comprising solid surface*antibody*antigen*enzyme-labeled antibody. Any of the conventional known orders of addition for the sandwich technique may be employed. For example, the antigen, immobilized antibody, and labeled antibody may be added simultaneously. For this technique, it is preferable to use a monoclonal antibody as the labeled antibody to minimize problems of cross blocking by immobilized and labeled antibodies.

In another technique, the "forward" sandwich assay, the antigen is reacted with the immobilized antibody prior to reacting with the labeled antibody. In this instance, after incubation of the antigen and immobilized antibody, the surface may be washed to remove unbound material prior to the addition of the labeled antibody.

In addition, the "reverse" sandwich assay may be used where the antigen and labeled antibody may be mixed, with or without an incubation step, prior to contact with the immobilized antibody. Researchers have suggested that this order of addition provides the advantages of increased sensitivity and decreased incubation times, as set forth in Piasso et al U.S. Patent No. 4,098,876.

After reaction to form the solid phase containing immobilized antibody-antigen-enzyme labeled antibody, the solid phase is separated from the solution containing the unreacted soluble enzyme-labeled antibody. This is preferably followed by a washing step to remove unbound labeled antibody from the surface of the solid phase.

Thereafter, the separated solid phase is contacted with a solution containing soluble substrate for the enzyme. An important feature of the invention is that the substrate is of a type which converts from soluble to insoluble form in the presence of the enzyme. Since the enzyme is on the surface of the solid phase, the insoluble substrate activated by the enzyme on the solid phase precipitates directly upon the solid phase. For this purpose, it is preferable to use as the solid phase a reaction surface of a dipstick which can be dipped into a test tube of substrate solution and mixed with the substrate.

The precipitated substrate is in a visually discernible colored form. In one embodiment, the substrate is initially uncolored and, in the presence of the enzyme, is converted to a colored form. In another embodiment, the soluble substrate is a first color or shade, and converts to a second color or shade upon precipitation.

After sufficient reaction time for the enzyme to cause the conversion of substrate to insoluble colored form, the solid phase is separated from the substrate solution. The presence of the visually discernible color on the solid phase is an indication that the biologically derived fluid contained the antigen to be detected. The intensity of the color on the solid phase is a semiquantitative determination of the concentration of antigen in the biological fluid. That is, the intensity of the color is proportional to the concentration of the antigen. By comparing the color on the solid phase with a color chart or reference solid phase such as dipstick surfaces, semiquantitative results may be obtained for the test results. Use of an instrument such as a reflectance densitometer with opaque dipsticks, or a spectrophotometer with an optically clear dipstick, can be used to enhance the quantitative aspect of this test system.

The antigen or antibody conjugate with the enzyme is preferably formed by conventional techniques of linkage by one or more covalent bonds. Such covalent linking may be accomplished by addition of external coupling or bridging molecules, or by direct condensation of existing side chains. Bifunctional bridging agents for accomplishing this purpose are well known in the art.

One advantage of the system is that the wash steps are particularly rapid in that the solid surface (dipstick) can be quickly rinsed after enzyme incubation and prior to contact with the substrate. Unbound enzyme is not as critical in this system in that enzyme-antibody which has not been fully washed off will diffuse into the substrate solution and not cause precipitation directly upon the solid surface. Thus, background noise from incomplete washing is minimized.

Another advantage of the system compared to reading the substrate solution is that it is easy to stop the enzyme-substrate reaction at the desired end point. That is, at an appropriate time the dipstick is removed from the enzyme solution, which stops the substrate reaction. This is to be contrasted with reading the solution wherein a chemical stop solution must be provided.

A further advantage to the system is that the colored dipstick may be used as a permanent record of test results so long as the substrate dye is of sufficient stability.

The above sandwich technique has been described with respect to the determination of an unknown antigen in a biologically derived solution using immobilized antibody and soluble enzyme-labeled antibody. The system is particularly effective for the detection of pregnancy where the antigen to be detected is human chorionic gonadotropin. In addition, the system can be used for the detection of ovulation where the antigen to be detected is luteinizing hormone. The system is also applicable to the determination of any antigen against which appropriate antibodies may be raised.

It should be understood that the sandwich technique is also applicable to the determination of antibody in such a system. In that instance, the antibody immobilized on the solid surface is substituted with immobilized antigen, and the enzyme-labeled antibody is substituted with an enzyme-labeled anti-immunoglobulin. In other respects, the aforementioned description is applicable.

One preferred final reacted product formed by the sandwich technique is a reacted dipstick for an immunoassay. In such dipstick, a solid reaction surface portion includes bound consecutive layers as follows. The first layer is antibody immunologically bound to antigen. Such antigen is, in turn, immunologically bound to enzyme-labeled antibody. A visually discernible colored substrate is precipitated onto the surface of such consecutive layers. The substrate is of a type which has been converted from a soluble to a precipitated colored form due to the presence of the enzyme. As set forth above, suitable antigens include human chorionic gonadotropin (hCG) and luteinizing hormone (hLH).

The present invention is also applicable to the so-called competitive binding technique for the colorimetric immunoassay of antigen to be detected in a biologically derived fluid sample. In this instance, the immobilized antibody is bound in a layer to a solid surface forming a solid phase in the same manner as set forth above. This solid phase is contacted with the biologically derived fluid containing antigen to be detected, and with free enzyme-labeled antigen of the same type as the antigen to be detected. A competitive immunological reaction is caused to occur between the immobilized antibody and both (a) the antigen to be detected, and (b) the enzyme-labeled antigen. That is, the concentration of antigen to be detected in the biologically derived fluid is inversely proportional to the enzyme-labeled antigen bound to the solid phase.

After the above competitive reaction, the solid phase from step (a) is separated from the liquid phase. Preferably, the surface is washed to remove unbound enzyme-labeled antigen from the surface.

Then, the separated solid phase is contacted with a solution containing soluble substrate for the enzyme, to cause the substrate to be converted into an insoluble form which deposits on the solid phase to impart a visually discernible color to it. The solid phase is separated from the substrate solution and is read as an inverse indication of the amount of antigen to be detected in the biologically derived sample. Thus, the color intensity on the solid surface increases with the decreasing presence of antigen to be detected.

The description of the above details of operation and advantages of the present invention with respect to the sandwich technique are also applicable to the analogous competitive technique.

An analogous reaction product for the competitive technique includes a reactive dipstick with a solid reaction surface portion. Bound to the surface portion is a layer of antibody, at least a portion of which is immunologically bonded to enzyme-labeled antigen, and visually discernible colored substrate precipitated onto the layer. The substrate is of a type which has been converted from a soluble to a precipitated colored form due to the presence of the enzyme.

As set forth above, suitable substrate solutions are of a type which generate an insoluble reaction product which precipitates on the dipstick or other solid surface. A particularly effective enzyme-substrate combination is the alkaline phosphatase enzyme and its substrate, 5-bromo-4-chloro-3-indolyl phosphate (commonly referred to as BCIP). This is a very stable phosphatase substrate which has been used as a histochemical stain for phosphatase activity in tissue sections. The enzymatic dephosphorylation of BCIP in amino-alcohol buffer yields the indolyl-3-ol derivative, which dimerizes to yield a blue colored, insoluble product.

Other substrates for alkaline phosphatase are set forth in the following Table 1:

## TABLE 1

| ENZYME | SUBSTRATE SOLUTION |
| --- | --- |
| ALKALINE PHOSPHATASES | (1)  Naphthol AS-MX phosphate[1] and Fast Blue RR Salt[2] (forms blue dye deposit) |
| | (2)  Naphthol AS-MX phosphate[1] and Fast Violet B Salt[3] (forms violet dye deposit) |

EP 0 125 118 B1

| ENZYME | | SUBSTRATE SOLUTION |
|---|---|---|

**ALKALINE PHOSPHATASES (cont'd)**  (3)  Naphthol AS-GR phosphate[4] and Fast Blue RR Salt[2] (forms green dye deposit)

(4)  5-bromo-4-chloro-3-indolyl-phosphate (forms blue deposit)

(5)  3-indoxyl phosphate (forms blue deposit)

**PEROXIDASES:**  (1)  p-phenylene diamine, catechol and $H_2O_2$ (forms brown black deposit)

(2)  3-amino-9-ethylcarbozole and $H_2O_2$ (forms reddish-brown deposit)

(3)  o-dianisidine hydrochloride and $H_2O_2$ (forms blue deposit)

**LACTATE DEHYDROGENASE:**  (1)  NADH (B-Nicotinamide adenine dinucleotide, reduced), phenazine methosulfate, and nitroblue tetrazolium (forms purple precipitate)

[1] 3-hydroxy-2-naphthoic acid, 2,4-dimethoxyanilide phosphate

[2] diazotized, 4-benzoylamino-2,5-dimethoxyaniline, $ZnCl_2$

[3] diazotized, 6-benzamido-4-methoxy-m-toluidine chloride

[4] 3-hydroxy-2-anthranoic acid 2-methylanilide

The following examples are illustrative of the present invention.

Example 1

Forming Dipsticks Covalently Coupled to Antibody

This example demonstrates the immobilization of an antibody to α-human luteinizing hormone on a 0.2 mm-thick sheet (the "sheet") of polyterephthalate ethylene glycol (Mylar) laminated with agar, and then forming a dipstick. The sheet is supplied under the trademark Gel Bond by FMC Bio Products, Rockland, Maine.

The sheet was soaked in water for 5 minutes and the agarose-coated portion to be coupled was soaked in a 20 mM aqueous solution of sodium meta-periodate for 30 minutes at 20-24°c. Following removal from the periodate solution, the sheet was washed three times in deionized water, then incubated overnight at 2-8°C in a buffer solution (0.1M $NaCO_3$, 0.5M NaCl, pH 9.2) containing 50 μg/ml of monoclonal antibody specific for α-subunit of human luteinizing hormone (hLH) and human chorionic gonadotropin (hCG) at 2-8°C. The antibodies were obtained by the hybridization of splenic lymphocytes with mouse myeloma cells, followed by cloning according to the procedure described by Kohler and Milstein in Nature, 256, 495-499 (1975). Incubation was followed by treatment of the sheet with an aqueous solution of sodium borohydride

7

(1.0 mg/ml) for 30 minutes at 4°C, and three washes with the phosphate buffered saline (PBS) solution mentioned above.

The reacted sheet contained a uniform layer of antibody permanently bonded to the sheet.

The sheets were dessicated overnight under vacuum and stored dry. The sheets were cut into 6 mm x 4 in. dipsticks using a paper cutter, and stored dessicated.

Example 2

Forming Dipsticks Containing Adsorbed Antibody

White polystyrene sheets 4 in. x 11-1/4 in. x 1/32 in., formed by extrusion from Dow-484 or Cosden 825E polystyrene washed free of oils and grease, were coated by immersion of the wide (11-1/4 in.) end into PBS containing 50 mM sodium phosphate, 0.9 w/v NaCl, pH 7.0, 5 $\mu$g/ml of monoclonal antibody specific for $\alpha$-subunit of hLH and hCG. The sheets were immersed to a height of 15 mm in the coating solution and incubated for approximately 24 hours at room temperature. Excess antibody was washed off the sheets by dipping the sheets into PBS, using three changes of PBS. The washed sheets were dessicated overnight under vacuum. The dry sheets were cut into 6 mm x 4 in. dipsticks using a paper cutter, and stored dessicated.

Example 3

Use of Dipsticks in Sandwich-Type Enzyme Immunoassay for hLH

This example demonstrates the use of the dipsticks prepared in Example 2 to develop a calibration curve for a non-competitive assay for hLH using "simultaneous" incubation of sample solid phase and labeled antibody.

A stock solution of monoclonal antibody specific for $\beta$-subunit of hLH was prepared according to the Kohler and Milstein procedure cited in Example 1. The antibodies were then conjugated to bovine intestinal alkaline phosphatase according to the procedure described by E. Engvall in Methods in Enzymology 70, 419-483 (1980). The resulting conjugate had an enzyme-to-antibody ratio of approximately 2:1, and the final conjugate solution had a concentration of approximately 36 ng/ml, based on active antibody diluted in 400 mm Tris HCl, pH 7.5, l mg/ml bovine serum albumin, 0.04% Tween-20 (polyoxyethylene sorbitan, mono-laurate), a non-ionic surfactant supplied by Atlas Chemical Industries, Inc., Wilmington, Delaware. A 10 mm x 75 mm culture tube was charged with 500 $\mu$l of this solution.

A solution (500 $\mu$l) of human urine spiked with a known amount of hLH was then added and the mixture was agitated. Seven such solutions of human urine were used with concentrations ranging from 0.0 to 7.0 ng/ml (0 to 60 mIU/ml), at equal 10 mIU/ml intervals, designated samples 1 through 7, respectively.

A dipstick measuring 4.0 in. in length and 6 mm in width, with a portion extending 1.5 cm from one end coated on both sides with the $\alpha$-antibody, was cut from the sheet prepared in Example 2. The dipstick was placed in the reaction tube with the antibody-coated portion fully submerged, and incubated thus at room temperature for thirty minutes. The dipstick was then removed and rinsed with a stream of tap water on both sides. Excess water was then shaken off and the dipstick was placed in 1.0 ml of a substrate solution consisting of 1.0 mg/ml of BCIP (5-Bromo, 4-Chloro, 3-Indolyl-Phosphate), and a suitable amino alcohol buffer, i.e., aminomethyl propanol, in a 10 mm x 75 mm culture tube at pH 9-11. The reaction was allowed to proceed at room temperature for 30 - 60 minutes, at which time the dipstick was removed from the substrate and air dried, after blotting or rinsing with water.

The results obtained from each of the seven spiked samples were as follows:

The sample 1 dipstick showed no color. The sample 2 dipstick showed a light blue color. The intensity of the color increased progressively from sample 2 through sample 7 in directed proportion to the hLH concentration. The hLH level of an unknown sample can be semiquantitated by visual comparison with the described reference dipsticks.

Example 4

Clear Mylar dipsticks were substituted for the dipsticks of Example 2 and used in the sandwich assay as described in Example 3. The blue dye deposited on the dipsticks was read spectrophotometrically by placing the colored end of the dipstick in the light path of a spectrophotometer. Where BCIP substrate was used the absorbence was read at 620 nm.

In a series of tests using a one-hour substrate incubation, the results of reading the clear dipsticks on a spectrophotometer are presented in the following table:

TABLE 2

| | hLH level(mIU/ml) | | | | | | | |
|---|---|---|---|---|---|---|---|---|
| | 0 | 20 | 30 | 40 | 50 | 60 | 100 | 150 |
| $A_{620}$ nm | .014 | .022 | .028 | .039 | .046 | .053 | .079 | .110 |

Example 5

Use of Dipsticks in Sandwich-Type Enzyme Immunoassay for hCG

This example demonstrates the use of the dipsticks prepared in Example 1 to develop a calibration curve for a non-competitive assay for hCG, using the "forward" sequential incubation of sample and solid-phase, and second incubation with labeled antibody.

A stock solution of monoclonal antibody specific for $\beta$-subunit of hCG was prepared according to the Kohler and Milstein procedure cited in Example 1. The antibodies were then conjugated to bovine intestinal alkaline phosphatase according to the procedure described by E. Engvall, cited in Example 3. The resulting conjugates had an enzyme-to-antibody ratio of approximately 2:1, and the final conjugate solution had a concentration of approximately 100 ng/ml, based on active antibody diluted in 50 mM Tris-HCl, .064% w/v NaCl, 1 mg/ml bovine serum albumin, pH 7.5,0.04% Tween-20.

A 10 mm x 75 mm culture tube was charged with 1 ml of this solution. A dipstick measuring 4 in. in length and 6 mm in width, with a portion extending 1.5 cm from one end coated on both sides with the $\alpha$-antibody, was cut from the sheet prepared in Example 2. The dipstick was placed in each of six urine samples containing 0 - 200 ng/ml hCG (0 - 1000 mIU/ml) with the antibody-coated portion fully submerged, and incubated at room temperature for 5 minutes. The dipstick was then removed from the urine sample and incubated for 5 to 10 minutes in the 10 mm x 75 mm culture tube charged with enzyme conjugate at room temperature. The dipstick was then removed and washed in distilled or tap water by swirling the dipstick in a beaker. Excess water was then shaken off and the dipstick was placed in 1.0 ml of substrate solution consisting of 1 mg/ml of BCIP and a suitable amino-alcohol buffer such as aminomethyl propanol, in a 10 mm x 75 mm culture tube at pH 9-11. The reaction was allowed to proceed at room temperature for 10 - 20 minutes, at which time the dipstick was removed from the substrate and air-dried, after blotting or rinsing with water.

The various levels of hCG in urine gave dipsticks with blue coloration directly proportional to the amount of hCG on the sample. The hCG negative urine sample gave a white or colorless dipstick. An unknown sample or a dilution of an unknown sample could be semi-quantitated by visual comparison with the described reference dipsticks. Using a single calibrator at the desired cut-off, qualitative results for pregnancy detection could be determined using this visual test. The test sensitivity could also be adjusted to set the visual detection limit to the desired cut-off for a qualitative test.

Example 6

This example demonstrates the use of the dipsticks prepared as in Example 2 to develop a calibration curve for a competitive enzyme immunoassay for mouse IgG.

Anti-mouse IgG (Fc specific) prepared by ammonium sulfate fractionation from rabbit antisera was used to coat polystyrene sheets as in Example 2.

Monoclonal mouse $IgG_1$ was coupled to bovine intestinal alkaline phosphatase as described in Example 3. This alkaline phosphatase coupled mouse $IgG_1$ was used as the labeled antigen in the following competitive EIA for mouse IgG, after dilution 1:2000 in Assay Buffer (50 mM Tris-HCl, pH 7.5, .064% NaCl, 0.04% Tween-20, 1 mg/ml bovine serum albumin).

A solution (100 $\mu$l) of mouse $IgG_1$ in phosphate buffered saline (10 mM sodium phosphate, pH 7.0, 0.9% NaCl) containing various levels of $IgG_1$, was added to a 10 mm x 75 mm culture tube charged with 900 $\mu$l of the labeled antigen in Assay Buffer, and the mixture was agitated. Six such solutions of mouse $IgG_1$ were used, with concentrations ranging from 0 to 0.1 mg/ml.

A dipstick measuring 4 in. in length and 6 mm in width, with a portion extending 2.0 cm from one end coated on both sides with anti-mouse IgG (Fc specific), was cut from the sheet prepared as described above. The dipstick was placed in the reaction tube with the antibody-coated portion fully submerged, and incubated at room temperature for 30 minutes. The stick was then removed and rinsed briefly in tap water on each side. Excess water was then shaken off, and the dipstick was placed in 1.0 ml of substrate solution consisting of 1.0 mg/ml BCIP in a suitable amino-alcohol buffer such as aminomethyl propanol, pH 9-11. The reaction was allowed to proceed at room temperature for 10 minutes, at which time the dipstick was removed from the substrate. The dipstick was air dried after gentle blotting.

There was an inverse relationship between blue color intensity on the dipstick and the level of mouse IgG in the sample. It is possible to semiquantitate an unknown sample or dilution of an unknown sample by visual comparison with the reference dipsticks.

**Claims**

1. A method for the colorimetric immunoassay of an immunologically reactive substance to be detected in a fluid sample, comprising:

contacting said sample with a non-swellable impermeable plastics continuous solid surface, whereby the chemical nature of the plastic surface is such as to permit binding of an antigen or an antibody to it, adapted to immobilize substance to be detected thereon from said sample and using said surface to immobilize an enzyme labeled diagnostic reagent for said substance to be detected in an amount related to the amount of said substance which has been immobilized, contacting the resulting solid material with a solution of a substrate for said enzyme which is converted thereby into an insoluble colored form and allowing said insoluble colored form to deposit on said resulting impermeable solid material as a precipitate to impart thereto a visually discernible color related to the presence of said substance, and separating the resulting colored solid material from said substrate solution.

2. A method as claimed in claim 1 which comprises:

(a) contacting said sample containing said substance to be detected with a non-swellable impermeable plastics continuous solid phase, whereby the chemical nature of the plastic surface is such as to permit binding of an antigen or an antibody to it, comprising immobilized anti-substance bound in a layer to said solid surface, and with either soluble enzyme-labeled anti-substance or with free enzyme-labeled substance of the same type as said substance to be detected thereby to cause an amount of the enzyme label to be immobilized which is related to the amount of said substance to be detected in the sample;

(b) separating the resulting solid phase from step (a) from liquid phase;

(c) contacting the separated solid phase from step (b) with a solution containing soluble substrate for the enzyme to cause the substrate to be converted into an insoluble colored form which deposits as a precipitate onto the non-swellable impermeable plastics continuous solid phase to impart a visually discernible color to it related to the presence of said substance to be detected in the sample; and

(d) separating said solid phase from said substrate solution.

3. A method as claimed in claim 2, wherein the immobilized anti-substance is a monoclonal antibody and/or an enzyme-labeled anti-substance is used which is an enzyme-labeled monoclonal antibody.

4. A method as claimed in any one of claims 1 to 3, wherein said substance to be detected is human chorionic gonadotropin, the method being used for the detection of pregnancy, or luteinizing hormone, the method being used for the detection of ovulation.

5. A method as claimed in any one of claims 1 to 4, wherein said substrate is uncolored prior to contact with the enzyme and converts to colored insoluble form in the presence of the enzyme.

6. A method as claimed in any one of claims 1 to 5, wherein said solid surface is a reaction surface portion of a plastic dipstick formed from a material selected from the group consisting of polymethylmethacrylate, polystyrene, polyethylene, polyterephthalate, polyester and polypropylene.

7. A method as claimed in any one of claims 1 to 6 which is a sandwich-type method for the colorimetric immunoassay of antigen to be detected in a biologically derived fluid sample using a solid surface, comprising the steps of:

EP 0 125 118 B1

(a) contacting said biologically derived fluid sample containing antigen to be detected with a non-swellable impermeable plastics continuous solid phase, whereby the chemical nature of the plastic surface is such as to permit binding of an antigen or an antibody to it, comprising immobilized antibody bound in a layer to a solid surface, and with soluble enzyme-labeled antibody being immunologically reactive with said antigen, to cause said antigen to bind immunologically to both said immobilized antibody and said labeled antibody on said solid phase;

(b) separating the impermeable solid phase from the solution containing unreacted soluble enzyme labeled antibody;

(c) contacting the separated solid phase from step (b) with a solution containing soluble substrate for the enzyme to cause the substrate to be converted into insoluble colored form which deposits as a precipitate onto the impermeable solid phase to impart a visually discernible color to it as an indication of the presence of the antigen to be detected in the biologically derived fluid sample; and

(d) separating said solid phase from said substrate solution.

8. A method as claimed in any one of claims 1 to 6 which is a competitive method for the colorimetric immunoassay of antigen to be detected in a biologically-derived fluid sample using a solid surface, comprising the steps of:

(a) contacting immobilized antibody found in a layer to a non-swellable impermeable plastics continuous solid surface, whereby the chemical nature of the plastic surface is such as to permit binding of an antigen or an antibody to it, forming a solid phase with said biologically derived fluid sample containing antigen to be detected and with free enzyme-labeled antigen of the same type as said antigen to be detected, to cause a competitive immunological reaction between said immobilized antibody and said antigen to be detected and enzyme-labeled antigen;

(b) separating the reacted solid phase of step (a) from the liquid phase;

(c) contacting the separated solid phase from step (b) with a solution containing soluble substrate for the enzyme to cause the substrate to be converted into insoluble colored form which deposits as a precipitate onto the impermeable solid phase to impart a visually discernible color to it as an inverse indication of the amount of the antigen to be detected in the biologically derived fluid sample; and

(d) separating said solid phase from said substrate solution.

9. A reacted dipstick for an immunoassay including an impermeable solid reaction surface portion to which is bound consecutive layers of antibody immunologically bonded to antigen which is immunologically bonded to enzyme-labeled antibody, and a visually discernible colored substrate precipitated onto said consecutive layers, said substrate having been converted from a soluble to a precipitated colored form due to the presence of said enzyme.

10. A reacted dipstick for an immunoassay including a non-swellable impermeable plastics continuous solid reaction surface portion, whereby the chemical nature of the plastic surface is such as to permit binding of an antigen or an antibody to it, to which is bound a layer of antibody, at least a portion of which is immunologically bonded to enzyme-labeled antigen, and visually discernible colored substrate precipitated onto said layer, said substrate having been converted from a soluble to a precipitated colored form due to the presence of said enzyme.

11. A reacted dipstick as claimed in claim 9 or claim 10, wherein the antigen is human chorionic gonadotropin or luteinzing hormone.

12. A process for forming a reacted dipstick as claimed in any one of claims 9 to 11 comprising carrying out the steps of a method as claimed in any one of claims 6 to 8.

**Patentansprüche**

1. Verfahren für das kolorimetrische Immunoessay einer immunologisch reaktiven, in einer Flüssigkeitsprobe zu entdeckenden Substanz, umfassend ein

Kontaktieren der besagten Probe mit einer aus nicht quellbaren, undurchlässigen Plastikmaterial bestehenden, durchgehenden, festen Oberfläche, wobei the chemische Natur der Plastikoberfläche derart ist, daß sie das Binden eines Antigens oder eines Antikörpers an ihr erlaubt, sowie geeignet ist, um darauf zu entdeckende Substanz von der besagten Probe zu immobilisieren, und ein Verwenden der besagten Oberfläche zum Immobilisieren eines Enzym markierten, diagnostischen Reagenz für die

11

besagte, zu entdeckende Substanz in einer Menge in bezug auf die Menge an besagter Substanz, die immobilisiert wurde, Kontaktieren des resultierenden festen Materials with einer Lösung eines Substrats für das besagte Enzym, das hierdurch in eine umlösliche, gefärbte Form konvertiert wird, und Ermöglichen der besagten unlöslichen, gefärbten Form, sich als ein Niederschlag auf dem besagten resultierenden, undurchlässigem, festen Material niederzuschlagen, um diesem a visuell unterscheidbare Farbe, die sich auf die Anwesenheit besagter Substanz bezieht, zu verleihen, und Trennen des resultierenden, gefärbten, festen Materials von der besagten Substratlösung.

2. Verfahren gemäß Anspruch 1, umfassend

(a) ein Kontaktieren der besagten die besagte zu entdeckende Substanz enthaltenden Probe mit einer nicht quellbaren, aus undurchlässigem Plastikmaterial bestehenden, kontinuierlichen, festen Phase, wobei die chemische Natur der Plastikfläche derart ist, daß sie ein Binden eines Antigens oder eines Antikörpers an ihr, umfassend immobilisierte, in einer Schicht an die besagte feste Oberfläche gebundene Antisubstanz, ermöglicht, und mit löslicher Enzym-markierter Antisubstanz oder mit freier Enzym-markierter Substanz des gleichen Typs wie die besagte zu entdeckende Substanz, um zu bewirken, daß eine Menge der Enzymmarkierung immobilisiert wird, die zur Menge der besagten, in der Probe zu entdeckenden Substanz in Bezug steht;
(b) ein Trennen der gemäß Schritt (a) resultierenden festen Phase von flüssiger Phase;
(c) ein Kontaktieren der abgetrennten festen Phase von Schritt (b) mit einer Lösung enthaltend lösliches Substrat für das Enzym, um zu bewirken, daß das Substrat in eine unlösliche, gefärbte Form konvertiert wird, die sich als Niederschlag auf der nicht quellbaren, aus undurchlässigem Plastik bestehenden, kontinuierlichen, festen Phase absetzt, um dieser eine visuell unterscheidbare Farbe bezüglich der Anwesenheit der besagten, in der Probe zu entdeckenden Substanz zu verleihen; und
(d) ein Trennen der besagten festen Phase von der besagten Substratlösung.

3. Verfahren gemäß Anspruch 2, wobei die immobilisierte Antisubstanz ein monoklonaler Antikörper ist und/oder eine enzymmarkierte Antisubstanz verwendet wird, die ein enzymmarkierter, monoklonaler Antikörper ist.

4. Verfahren gemäß einem der Ansprüche 1 bis 3, wobei die besagte zu entdeckende Substanz menschliches Choriongonadotropin ist, wobei das Verfahren zur Schwangerschaftsfeststellung verwendet wird, oder luteinisierendes Hormon ist, wobei das Verfahren zur Ovulationsfeststellung verwendet wird.

5. Verfahren gemäß einem der Ansprüche 1 bis 4, wobei das besagte Substrat vor dem Kontakt mit dem Enzym farblos ist und sich in eine gefärbte unlösliche Form in Anwesenheit des Enzyms umwandelt.

6. Verfahren gemäß einem der Ansprüche 1 bis 5, wobei die besagte feste Oberfläche ein Reaktionsflächenteil eines Eintauchstabes gebildet aus einem Material ausgewählt aus der Gruppe umfassend Polymethylmethacrylat, Polystyrol, Polyethylen, Polyterephthalat, Polyester und Polypropylen ist.

7. Verfahren gemäß einem der Ansprüche 1 bis 6, das ein sandwichartiges Verfahren für das kolorimetrische Immunoessay eines in einer biologisch erhaltenen Flüssigkeitsprobe enthaltenen Antigens unter Verwendung einer festen Oberfläche ist, umfassend die Schritte des

(a) Kontaktierens der besagten biologisch erhaltenen Flüssigkeitsprobe, die das zu entdeckende Antigen enthält, mit einer nicht quellbaren, aus undurchlässigem Plastikmaterial bestehenden, kontinuierlichen, festen Phase, wobei die chemische Natur der Plastikoberfläche derart ist, daß sie ein Binden eines Antigens oder eines Antikörpers auf ihr ermöglicht, umfassend immobilisierten Antikörper gebunden in einer Schicht an einer festen Oberfläche, und mit löslichem, enzymmarkiertem, immunologisch mit dem besagten Antigen reaktiven Antikörper, um zu bewirken, daß das besagte Antigen immunologisch sowohl an dem besagten immobilisierten Antikörper als auch an dem besagten markierten Antikörper auf der besagten festen Phase gebunden wird;
(b) Trennens der undurchlässigen festen Phase von der Lösung enthaltend unreagierten, löslichen, enzymmarkierten Antikörper;
(c) Kontaktierens der abgetrennten festen Phase aus Schritt (b) mit einer Lösung enthaltend lösliches Substrat für das Enzym, um zu bewirken, daß das Substrat in eine unlösliche, gefärbte Form umgewandelt wird, die sich als Niederschlag auf der undurchlässigen festen Phase absetzt, um dieser eine visuell unterscheidbare Farbe als eine Indikation für die Anwesenheit des in der biologisch erhaltenen Flüssigkeitsprobe zu entdeckenden Antigens zu verleihen; und

(d) Trennens der besagten festen Phase von der besagten Substratlösung.

8. Verfahren gemäß einem der Ansprüche 1 bis 6, das ein konkurrierendes Verfahren für das kalorimetrische Immunoessay eines in einer biologisch abgeleiteten Flüssigkeitsprobe enthaltenen Antigens ist, das eine feste Oberfläche verwendet, umfassend die Schritte des

(a) Kontaktierens von immobilisiertem, in einer Schicht auf einer nicht quellbaren, aus undurchlässigem Plastikmaterial bestehenden, kontinuierlichen, festen Oberfläche befindlichem Antikörper, wobei die chemische Natur der Plastikfläche derart ist, daß sie das Binden eines Antigens oder eines Antikörpers an ihr erlaubt, das eine feste Phase mit der besagten biologisch abgeleiteten, zu entdeckendes Antigen enthaltenden Flüssigkeitsprobe und mit freiem enzymmarkiertem Antigen des gleichen Typs wie das besagte, zu entdecken Antigen bildet, um eine konkurrierende immunologische Reaktion zwischen dem besagten immobolisierten Antikörper und dem besagten, zu entdeckenden Antigen und enzymmarkierten Antigen zu bewirken;

(b) Trennens der reagierten festen Phase von Schritt (a) von der flüssigen Phase;

(c) Kontaktierens der separierten festen Phase von Schritt (b) mit einer Lösung enthaltend lösliches Substrat für das Enzym, um zu bewirken, daß das Substrat in eine unlösliche, gefärbte Form überführt wird, die sich als Niederschlag auf der undurchlässigen, festen Phase absetzt, um dieser eine visuell unterscheidbare Farbe als eine Umkehrindikation für die Menge des in der biologisch abgeleiteten Flüssigkeitsprobe enthaltenen Antigens zu verleihen; und

(d) Trennens der besagten festen Phase von der besagten Substratlösung.

9. Reagierter Eintauchstab für ein Immunoessay, umfassend einen undurchlässigen festen Reaktionsoberflächenabschnitt, an den aufeinanderfolgende Schichten von Antikörper immunologisch gebunden an Antigen, das immunologisch an enzymmarkierten Antikörper gebunden ist, und ein visuell unterscheidbar gefärbtes, auf besagte aufeinanderfolgende Schichten niedergeschlagenes Substrat, das von einer löslichen in eine niedergeschlagene gefärbte Form aufgrund der Anwesenheit des besagten Enzyms umgewandelt wurde.

10. Reagierter Eintauchstab für ein Immunoessay umfassend einen nicht quellbaren, aus undurchlässigem Plastikmaterial bestehenden, kontinuierlichen, festen Reaktionsoberflächenabschnitt, wobei die chemische Natur des Plastikoberfläche derart ist, daß sie es erlaubt, ein Antigen oder einen Antikörper an ihr zu binden, an dem eine Schicht aus Antikörper, von dem wenigstens ein Teil immunologisch an enzymmarkiertes Antigen gebunden ist, und visuell unterscheidbar gefärbtes Substrat, das auf die besagte Schicht niedergeschlagen ist, gebunden ist, wobei das besagte Substrat von einer löslichen in eine niedergeschlagene gefärbte Form aufgrund der Anwesenheit des besagten Enzyms konvertiert worden ist.

11. Reagierter Eintauchstab gemäß Anspruch 9 oder 10, wobei das Antigen menschliches Choriongonadotropin oder luteinisierendes Hormon ist.

12. Verfahren zur Herstellung eines reagierten Eintauchstabs gemäß einem der Ansprüche 9 bis 11, umfassend das Ausführen der Schritte eines Verfahrens wie in einem der Ansprüche 6 bis 8 beansprucht.

**Revendications**

1. Procédé pour l'essai immunologique colorimétrique d'une substance immunologiquement réactive à déceler dans un échantillon liquide, comprenant les étapes consistant à : mettre ledit échantillon en contact avec une surface solide non gonflable, imperméable, plastique et continue, la nature chimique de la surface plastique étant telle qu'elle permet à un antigène ou à un anticorps de s'y fixer, conçue pour immobiliser une substance à y déceler dans ledit échantillon, et utiliser ladite surface pour immobiliser un réactif de diagnostic marqué enzymatiquement pour ladite substance à déceler, en une quantité qui est fonction de la quantité de ladite substance qui a été immobilisée, mettre le produit solide résultant en contact avec une solution d'un substrat pour ladite enzyme, qui est transformé ainsi en une forme colorée insoluble, et laisser ladite forme colorée insoluble se déposer sur ledit produit solide imperméable résultant, sous la forme d'un précipité, pour lui conférer une couleur discernable

13

visuellement en fonction de la présence de ladite substance, et séparer le produit solide coloré résultant de ladite solution de substrat.

2. Procédé selon la revendication 1, qui comprend les étapes consistant à :

(a) mettre ledit échantillon contenant ladite substance à déceler en contact avec une phase solide non gonflable, imperméable, plastique et continue, la nature chimique de la surface plastique étant telle qu'elle permet à un antigène ou à un anticorps de s'y fixer, comprenant une anti-substance immobilisée fixée en couche sur ladite surface solide, et avec une anti-substance soluble marquée enzymatiquement ou avec une substance libre marquée enzymatiquement, du même type que ladite substance à déceler, pour provoquer l'immobilisation d'une quantité de marqueur enzymatique qui est fonction de la quantité de ladite substance à déceler dans l'échantillon ;

(b) séparer la phase solide résultante de l'étape (a) de la phase liquide ;

(c) mettre la phase solide séparée de l'étape (b) en contact avec une solution contenant un substrat soluble pour que l'enzyme oblige le substrat à se transformer en une forme colorée insoluble qui se dépose sous la forme d'un précipité sur la phase solide, non-gonflable, imperméable, plastique et continue, pour lui conférer une couleur discernable visuellement, qui est fonction de la présence de ladite substance à déceler dans l'échantillon ; et

(d) séparer ladite phase solide de ladite solution de substrat.

3. Procédé selon la revendication 2, dans lequel l'anti-substance immobilisée est un anticorps monoclonal, et/ou on utilise une anti-substance marquée enzymatiquement, qui est un anticorps monoclonal marqué enzymatiquement.

4. Procédé selon l'une quelconque des revendications 1 à 3, dans lequel ladite substance à déceler est la chorio-gonadotrophine humaine quand le procédé est mis en oeuvre pour la détection de la grossesse, ou l'hormone lutéinisante quand le procédé est mis en oeuvre pour la détection de l'ovulation.

5. Procédé selon l'une quelconque des revendications 1 à 4, dans lequel ledit substrat n'est pas coloré avant d'être mis en contact avec l'enzyme et se transforme en une forme colorée insoluble en présence de l'enzyme.

6. Procédé selon l'une quelconque des revendications 1 à 5, dans lequel ladite surface solide est une partie de surface de réaction d'un bâtonnet en plastique formé à partir d'un matériau choisi dans le groupe comprenant le polyméthylméthacrylate, le polystyrène, le polyéthylène, le polytéraphtalate, le polyester et le polypropylène.

7. Procédé selon l'une quelconque des revendications 1 à 6, qui est un procédé du type sandwich pour l'essai immunologique colorimétrique d un antigène à déceler dans un échantillon liquide d'origine biologique au moyen d'une surface solide, comprenant les étapes consistant à :

(a) mettre ledit échantillon liquide d'origine biologique contenant l'antigène à déceler en contact avec une phase solide, non gonflable, imperméable, plastique et continue, la nature chimique de la surface plastique étant telle qu'elle permet à un antigène ou à un anticorps de s'y fixer, comprenant un anticorps immobilisé fixé en couche sur une surface solide, et avec un anticorps soluble marqué enzymatiquement et capable de réagir immunologiquement avec ledit antigène, pour obliger ledit antigène à se fixer immunologiquement à la fois sur ledit anticorps immobilisé et sur ledit anticorps marqué sur ladite phase solide ;

(b) séparer la phase solide imperméable de la solution contenant l'anticorps soluble marqué enzymatiquement n'ayant pas réagi ;

(c) mettre la phase solide séparée de l'étape (b) en contact avec une solution contenant un substrat soluble pour que l'enzyme oblige le substrat à se transformer en une forme colorée insoluble qui se dépose sur la phase solide imperméable sous la forme d un précipité, pour lui conférer une couleur discernable visuellement, comme indication de la présence de l'antigène à déceler dans l'échantillon liquide d'origine biologique ; et

(d) séparer ladite phase solide de ladite solution de substrat.

8. Procédé selon l'une quelconque des revendications 1 à 6, qui est un procédé compétitif pour l'essai immunologique colorimétrique d'un antigène à déceler dans un échantillon liquide d'origine biologique, au moyen d une surface solide, comprenant les étapes consistant à :

(a) mettre l'anticorps immobilisé fixé en couche sur une surface solide, non gonflable, imperméable, plastique et continue, la nature chimique de la surface plastique étant telle qu'elle permet à un antigène ou à un anticorps de s'y fixer, formant une phase solide, en contact avec ledit échantillon liquide d'origine biologique contenant l'antigène à déceler et avec un antigène libre marqué enzymatiquement, du même type que ledit antigène à déceler, pour déclencher une réaction immunologique de compétition entre ledit anticorps immobilisé et ledit antigène à déceler et l'antigène marqué enzymatiquement ;

(b) séparée la phase solide ayant réagi de l'étape (a) de la phase liquide ;

(c) mettre la phase solide séparée de l'étape (b) en contact avec une solution contenant un substrat soluble pour que l'enzyme provoque la transformation du substrat en une forme colorée insoluble qui se dépose sur la phase solide imperméable sous la forme d'un précipité, pour lui conférer une couleur discernable visuellement, en tant qu'indication inverse de la quantité d'antigène à déceler dans l'échantillon liquide d'origine biologique ; et

(d) séparer ladite phase solide de ladite solution de substrat.

9. Bâtonnet d'essai immunologique ayant subi une réaction, comprenant une partie formant une surface de réaction solide, sur laquelle sont fixées des couches consécutives d'anticorps lié immunologiquement à un antigène qui est lié immunologiquement à un anticorps marqué enzymatiquement, et un substrat coloré de façon visuellement discernable, précipité sur lesdites couches consécutives, ledit substrat ayant été transformé d'une forme soluble en une forme précipitée colorée, en raison de la présence de ladite enzyme.

10. Bâtonnet d'essai immunologique ayant subi une réaction, comprenant une partie formant une surface de réaction solide, non gonflable, imperméable, plastique et continue, la nature chimique de la surface plastique étant telle qu'elle permet à un antigène ou à un anticorps de s'y fixer, à laquelle est fixée une couche d'anticorps, dont au moins une partie est liée immunologiquement à un antigène marqué enzymatiquement, et un substrat coloré de façon visuellement discernable, précipité sur ladite couche, ledit substrat ayant été transformé d'une forme soluble en une forme précipitée colorée, en raison de la présence de ladite enzyme.

11. Bâtonnet d'essai ayant subi une réaction, selon la revendication 9 ou 10, où l'antigène est la choriogonadotrophine humaine ou l'hormone lutéinisante.

12. Procédé pour former un bâtonnet ayant subi une réaction selon l'une quelconque des revendications 9 à 11, consistant à mettre en oeuvre les étapes d'un procédé selon l'une quelconque des revendications 6 à 8.